# EUROPEAN PATENT SPECIFICATION

(11) **EP 2 796 538 B1**
(45) Date of publication and mention of the grant of the patent: **19.04.2017**
(21) Application number: 11878294.5
(22) Date of filing: 20.12.2011
(51) Int. Cl.: C12M 1/34, C12Q 1/02

(54) **OBJECT SELECTING DEVICE AND OBJECT SELECTING METHOD**
OBJEKTAUSWAHLVORRICHTUNG UND VERFAHREN ZUR OBJEKTAUSWAHL
DISPOSITIF DE SÉLECTION D'OBJETS ET PROCÉDÉ DE SÉLECTION D'OBJETS

(43) Date of publication of application: 29.10.2014
(73) Proprietor: Yamaha Hatsudoki Kabushiki Kaisha, Shizuoka-ken 438-8501 (JP)
(72) Inventor: ITO, Saburo, Iwata-shi Shizuoka-ken 438-8501 (JP)
(74) Representative: Müller-Boré & Partner Patentanwälte PartG mbB
(86) International application number: PCT/JP2011/007115
(87) International publication number: WO 2013/093961

(56) References cited:
- EP-A1- 2 796 538
- WO-A1-01/96019
- WO-A1-2011/103143
- WO-A2-2007/022026
- JP-A- 2004 503 389
- JP-A- 2004 510 996
- JP-A- 2007 504 816
- JP-A- 2008 504 816
- JP-A- 2009 504 161
- US-A1- 2006 128 006
- YULI WANG ET AL: "Micromolded arrays for separation of adherent cells", LAB ON A CHIP, vol. 10, no. 21, 1 January 2010 (2010-01-01), page 2917, XP055156586, ISSN: 1473-0197, DOI: 10.1039/c0lc00186d

## Description

The present invention relates to an object selecting device and an object selecting method. More specifically, the present invention relates to an object selecting device and an object selecting method for selecting only a selection object of a predetermined shape from a collection of objects having different shapes.

Conventionally, in various fields, sieving devices have been used to select particles according to size or outer shape (hereinafter, these are referred to merely as shape). Larger particles to be selected include tablets, capsules and granulated granules and smaller ones include bio-based cells used in the fields of bio-related technology and medicine.

If, for example, cells are selected to make the shape uniform in this way, deviations of test conditions can be made smaller in various tests using such cells. The selected cells can be subjected to high-throughput screening (HTS) and the like.

However, an operation of selecting only objects shaped to be suitable for a test not only from tablets and capsules, but also from a plurality of cells having various shapes is very difficult.

In view of such a problem, patent literature 1 discloses a method for manufacturing a platen having a desired thickness and formed with a plurality of through holes. The platen of patent literature 1 includes the plurality of through holes and selects cells by supporting cells and the like in the through holes. Further, patent literature 2 discloses a selecting device for selecting capsules dyed with a dye. The selecting device of patent literature 2 includes a holding container having a mesh in which the capsules are to be arranged and a pipette for causing only the capsules satisfying conditions to pass through mesh openings by giving a water flow and selecting only such capsules by suction.

In this context, WO 2007/022026 A2 describes methods of making devices having a high-density array of through-holes, methods of cleaning and refurbishing the same, methods of making high-density arrays of chemical, biochemical and biological compounds, as well as methods of analyzing the contents of such arrays.

Patent literature 1: Japanese Unexamined Patent Publication (Japanese translation of PCT application) No. 2009-504161
Patent literature 2: Japanese Unexamined Patent Publication No. H05-103658

However, in the case of sucking and extracting cells supported by the platen formed with the through holes disclosed in patent literature 1 or capsules supported on the mesh disclosed in patent literature 2 by a selection nozzle, there is a problem that some of the cells or the capsules are sucked by the nozzle to be distorted and deformed and, at times, destroyed. Particularly, in the case of bio-based objects having a soft property such as cells, some of them may possibly become dead cells due to a charge in the property caused by deformation.

The present invention was developed in view of such a conventional problem and aims to provide an object selecting device and an object selecting method capable of selecting only a selection object having a predetermined shape from a collection of objects having different shapes without deformation or destruction.

An object selecting device according to one aspect of the present invention is an object selecting device for selecting a selection object from a collection of objects including the selection object, comprising:
a container including an inner bottom part and configured to store liquid,
a plate having a top surface and a bottom surface, including a plurality of through holes at a support position for the selection object, each of the through holes penetrating from the top surface to the bottom surface, and to be immersed in the liquid stored in the container,
a determining means arranged below the container and including a microscope for observing from below the container whether or not the object supported at the support position is desired, and
a removing means arranged above the container and configured to remove the object determined to be defective by the determining means and leaving only the selection object in a state supported at the support position, characterized in that:
   the through hole includes tapered walls that form pointed portions between tapered portions of the through hole such that when an object immersed in a liquid precipitates under gravity, it is brought into contact with the inner wall surface of the through hole, and an opening area at the upper end of the tapered portion is larger than an opening area at the lower end of the tapered portion,
   the determining means observes the objects supported at the support position, the supported objects having a size allowing to be introduced into the through hole from the upper ends of the tapered portions while not allowing to pass through the lower ends of the tapered portions, for determining whether or not the objects are desired, and
   the removing means removes the objects determined to be defective by the determining means out of the objects supported by the inner wall surface.

An object selecting method according to another aspect of the present invention is a method for selecting a selection object from a collection of objects including the selection object, by using the object selecting device of the present invention, comprising:
an immersion step of immersing the plate in the container storing liquid,
a precipitation step of adding a collection of objects including the selection object to the liquid from a side of the top surface of the plate and causing the collection of objects to precipitate along a direction of gravity into the through hole,
an arrangement step of supporting objects having a size allowing to be introduced into the through hole from the upper ends of the tapered portions while not allowing to pass through the lower ends of the tapered portions out of the collection of objects precipitating in the through hole at the support position,
a determination step of determining from below the container by a microscope included in a determining means whether or not the object supported at the support position is desired, and
a removal step of removing the object detected to be defective by the determining means by a removing means arranged above the container and leaving only the selection object in a state supported at the inner wall surface out of the objects supported by the support position.

An object, features and advantages of the present invention will become more apparent upon reading the following detailed description along with the accompanying drawings.

The figures show:
FIG. 1 is a diagram showing the configuration of an object selecting device of a first embodiment of the present invention,
FIG. 2 is a perspective view showing a through hole of the first embodiment of the present invention,
FIGS. 3 are diagrams showing a state of selecting selection objects in the object selecting device of the first embodiment of the present invention,
FIG. 4 is a perspective view showing a state where selection objects are supported by a plate of the first embodiment of the present invention,
FIGS. 5 are diagrams showing a state of operation of push-out mechanisms of the first embodiment of the present invention,
FIG. 6 is a diagram showing a state of operation of a push-out mechanism of a second embodiment of the present invention,
FIG. 7 is a diagram showing a state of operation of a push-out mechanism of a third embodiment of the present invention,
FIG. 8 is a diagram showing a state of operation of a pull-out mechanism of a fourth embodiment of the present invention,
FIG. 9 is a diagram showing a state of operation of another example of the pull-out mechanism of the fourth embodiment of the present invention,
FIG. 10 is a diagram showing each step of an object selecting method of the present invention,
FIG. 11 is a micrograph of a cell aggregate having a distorted shape, and
FIG. 12 is a micrograph of a cell aggregate with an uneven density.

### [Object Selecting Device]

### (First Embodiment)

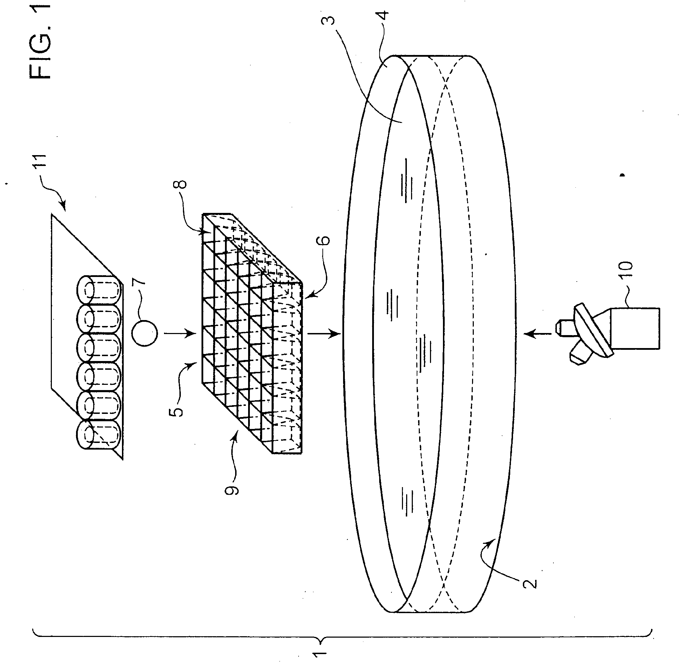

Hereinafter, an object selecting device 1 of a first embodiment of the present invention is described in detail with reference to the drawings. FIG. 1 is a diagram showing the configuration of the object selecting device 1 of the first embodiment of the present invention.

The object selecting device 1 of this embodiment includes a container 4 including an inner bottom part 2 and configured to store liquid 3, a plate 9 having a top surface 5 and a bottom surface 6, including through holes 8 at support positions for selection objects 7 and to be immersed in the liquid 3 stored in the container 4, a phase-contrast microscope 10 (determining means) for determining whether or not the objects supported at the support positions are good, and push-out mechanisms 11 (removing means) for removing the object determined to be defective by the phase-contrast microscope 10. Each component is described below.

### <Concerning Selection Object 7>

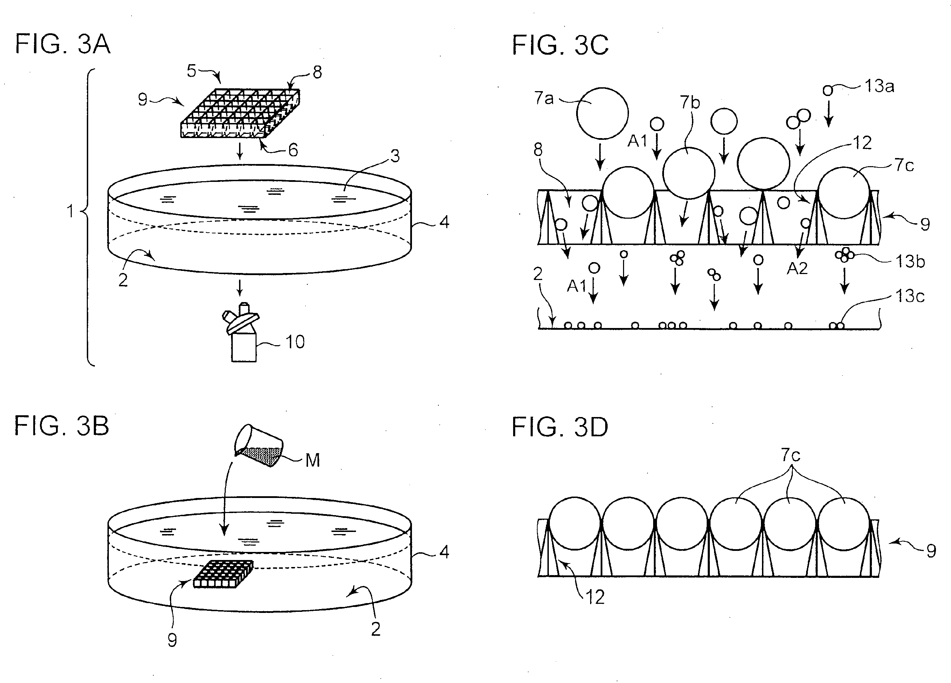

The selection objects 7 are objects to be selected from a collection of objects M (see FIG. 3B) using the object selecting device 1 of this embodiment.

The type of the collection of objects M is not particularly limited, but examples thereof, include mixtures of particles having various shapes and particle diameters, cell culture solutions and cell treatment solutions containing cells and impurities having various sizes. For example, if the collection of objects M is mixed slurry of particles having various shapes and particles having a predetermined shape are selected using the object selecting device 1, the selected particles having the predetermined shape fall under the selection objects 7. Similarly, if the collection of objects M is a cell culture solution or a cell treatment solution including cells and impurities having various sizes and only cells having a predetermined shape are selected using the object selecting device 1, the selected sells having the predetermined shape fall under the selection objects 7.

The selection objects are preferably bio-based cells, more preferably bio-based cell aggregates.

Bio-based cells are objects having relatively large shape deviations. Thus, if the selection objects 7 are bio-based cells, cells having a uniform shape can be selected by using the object selecting device 1 of this embodiment. This can largely contribute to improved operation efficiency in the fields of bio-related technology and medicine.

If the selection objects 7 are bio-based cell aggregates (spheroids), a result considering functions of individual cells can be obtained as compared with a test result obtained using one cell since a biosimilar environment considering interactions among cells is reconfigured in the cell aggregate, and experiment conditions can be made uniform in accordance with an environment in a biological body. Thus, by using the object selecting device 1 of this embodiment, a highly reliable result can be obtained in the fields of bio-related technology and medicine.

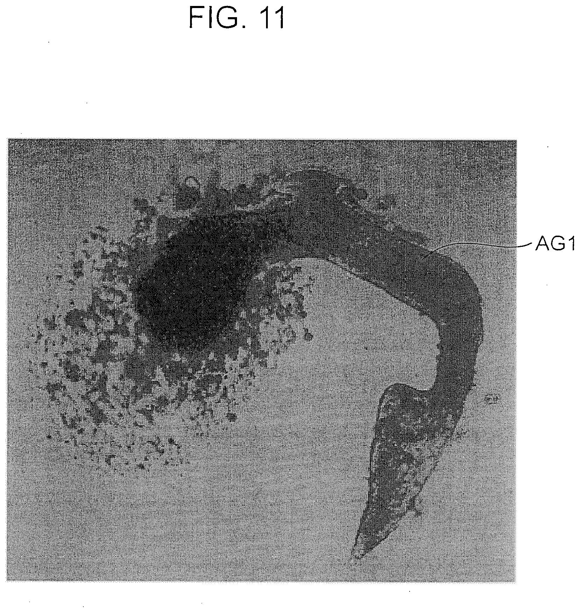

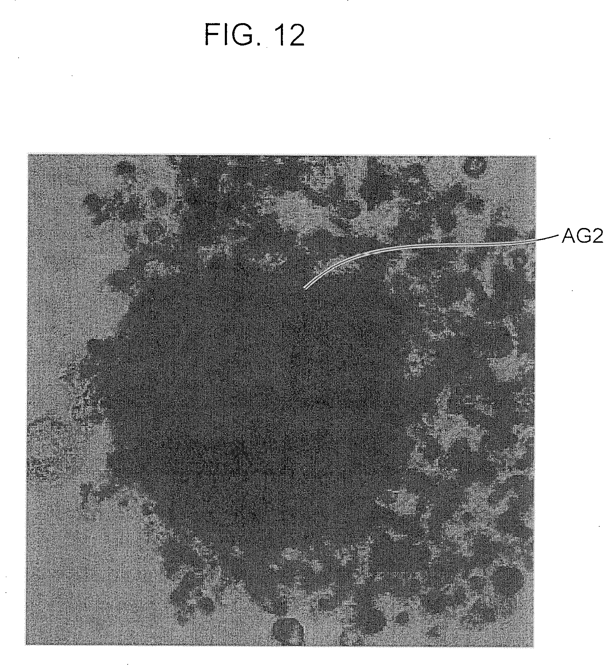

Here, such a cell aggregate is generally formed by aggregating several to several hundred thousands of individual cells. Thus, cell aggregates vary in size. A cell aggregate formed by living cells has a substantially spherical shape. However, if some of cells constituting a cell aggregate are altered or dead cells, the cell aggregate may become a cell aggregate AG1 having a distorted shape as shown in FIG. 11 or a cell aggregate AG2 with an uneven density as shown in FIG. 12. By using the object selecting device 1 of this embodiment, only cell aggregates having a shape suitable for a test can be selected from a plurality of cell aggregates having these various shapes.

### <Concerning Container 4>

The container 4 stores the liquid 3. In FIG. 1, the container 4 is illustrated to be formed of a bottomed cylindrical body including the inner bottom part 2 and having an open upper end.

The shape of the container 4 is not particularly limited, but the inner bottom part 2 is preferably flat and a relatively flat shape whose height is relatively smaller than a width is preferably adopted in terms of operability, stability and the like.

The container 4 has only to have such a size sufficient to store the liquid 3 to such an extent that the plate 9 to be described later can be completely immersed.

The material of the container 4 is not particularly limited, but it is preferable to use a translucent material in terms of possibility to easily confirm a state of a content stored in the container 4. Further, if both the container 4 and the plate 9 are made of a translucent material as described later, operation efficiency can be improved since a user can continuously observe the selection objects 7 using the phase-contrast microscope 10 to be described later from below the container 4.

The translucent material is not particularly limited, but it is preferable to use, for example, thermoplastic resins, thermosetting resins and photocurable resins. More specifically, the examples of the translucent material include polyethylene resins; polyethylene naphthalate resins; polypropylene resins; polyimide resins; polyvinyl chloride resins; cycloolefin copolymers; norbornene-containing resins; polyether sulfone resins; polyethylene naphthalate resins; cellophanes; aromatic polyamide resins; (meth)acrylic resins such as polymethyl (meth)acrylates; styrene resins such as polystyrenes and styrene-acrylonitrile copolymers; polycarbonate resins; polyester resins; phenoxy resins; butyral resins; polyvinyl alcohols; cellulose-based resins such as ethyl cellulose, cellulose acetate and cellulose acetate butyrate; epoxy resins; phenol resins; silicone resins; and polylactic acids.

It is also preferable to use inorganic materials such as metal alkoxides, ceramic precursor polymers, solutions obtained through hydrolysis polymerization of solutions containing metal alkoxides by a sol-gel method, or inorganic materials obtained by solidifying combinations of these such as inorganic materials having a siloxane bond (polydimethylsiloxane, etc.) and glass.

Soda glass, quartz, borosilicate glass, Pyrex (R) glass, low melting point glass, photosensitive glass and other optical glasses having various refractive indices and Abbe numbers can be widely used as the glass.

A circular glass dish having a height of several mm to several cm and a diameter of about 10 cm can be used as the container 4 satisfying these conditions.

The liquid 3 stored in the container 4 is not particularly limited if it does not degrade properties of the selection objects 7, and can be appropriately selected according to the type of the selection objects 7. Typical examples of the liquid 3 may include, for example, cell freezing solutions such as glycerol to be added before refrigeration storage and Cell Bankers (produced by Juji Field Inc.), formalin, reagents for fluorescent staining, antibodies, purified water and physiological saline solution in addition to media such as basic media, synthetic media, Eagle's media, RPMI media, Fischer's media, Ham's media, MCDB media and serums. If the selection objects 7 are cells, a culture preservation solution suited to the cells can be used. For example, in the case of using BxPC-3 (human pancreatic tumor cells), which are bio-based cells, as the selection objects 7, a mixture of a RPMI-1640 medium with 10 % of FBS (Fetal Bovine Serum), to which a supplement such as antibiotic or sodium pyruvate is added if necessary, can be used as the liquid 3.

The amount of the liquid 3 stored in the plate 9 is not particularly limited and is preferably sufficient to completely immerse the plate 9 to be described later.

### <Concerning Plate 9>

The plate 9 is used by being immersed in the liquid 3 stored in the container 4. The plate 9 includes through holes 8 at support positions where the selection objects 7 are supported. The plate 9 illustrated in FIG. 1 has a flat rectangular parallelepipedic shape having the top surface 5 and the bottom surface 6, and a plurality of through holes 8 penetrating from the top surface 5 to the bottom surface 6 are arranged in a 6x6 matrix, i.e. a total of thirty-six through holes 8 are arranged.

The shape of the plate 9 is not particularly limited, but is preferably a flat shape because the plate 9 is easily immersed in the container 4 and the selection objects 7 are easily selected from the collection of objects M precipitated right below with gravity when the container 4 has a flat shape and because a microscope is easily focused in observing the objects supported at the support positions of the plate 9.

The plate 9 has only to have such a size with a width smaller than an opening width of the container 4 and a height smaller than a storage depth of the container 4 since the plate 9 needs to be immersed in the liquid 3 stored in the container 4.

The material of the plate 9 is not particularly limited, but a translucent material is preferably used because a state of a content can be easily confirmed. Further, as described later, if both the container 4 and the plate 9 are made of a translucent material, the user can continuously observe the selection objects 7 using the phase-contract microscope 10 to be described later from above or below the container 4.

The translucent material is not particularly limited, but the materials described in the description of the container 4 can be used.

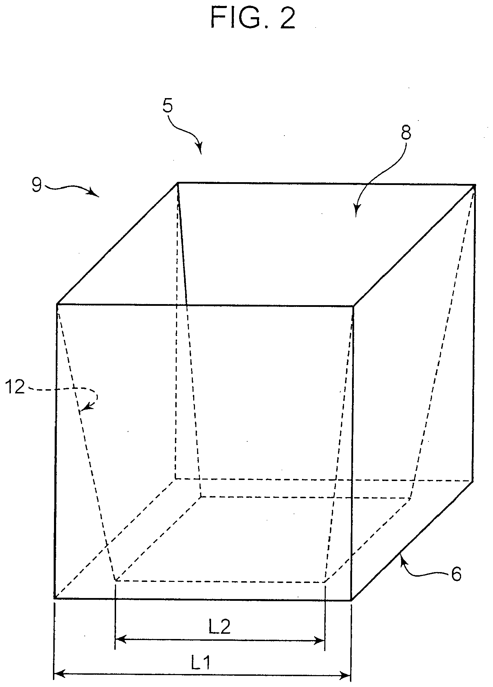

The plate 9 of this embodiment includes the through holes 8 vertically penetrating through the top surface 5 and the bottom surface 6 of the plate 9 at the support positions for the selection objects 7 as shown in FIGS. 1 and 2. FIG. 2 is a perspective view showing the through hole 8 of the first embodiment of the present invention.

The through hole 8 has a tapered portion 12. The tapered portion 12 is provided to allow the selection object 7 to precipitate along a direction of gravity and support the selection object 7 in contact with the inner wall surface of the through hole 8 in a state where the plate 9 is immersed in the liquid 3 in the container 4. An opening area at an upper end side of the tapered portion 12 is larger than that at a lower end side of the tapered portion 12. Specifically, the tapered portion 12 is so formed that the opening area is gradually narrowed from the top surface 5 toward the bottom surface 6.

A procedure of selecting the selection objects 7 is described in detail later. The through holes 8 of the plate 9 capture only the selection objects 7 (selection objects 7c) by the tapered portions 12 and allow non-objects 13 (non-objects 13 c) to pass and precipitate to the inner bottom part 2 of the container 4 as shown in FIGS. 3C and 3D.

In FIG. 3C, reference sign 7a denotes a selection object precipitating in a direction of gravity A1 and reference sign 13a denotes a non-object precipitating in the direction of gravity A1. Reference sign 7b denotes a selection object precipitating in the through hole 8 and reference sign A2 denotes a direction of precipitation of the selection object 7b along the tapered portion 12. Since diameters of the non-objects are smaller than the opening area at the lower ends of the tapered portions 12, the non-objects pass through the through holes 8. Reference sign 13b denotes a non-object precipitating in the direction of gravity after passing through the through hole 8. The non-objects 13b having passed through the through holes 8 precipitate to the inner bottom part 2 of the container. Reference sign 13c denotes a non-object precipitated on the inner bottom part 2 of the container 4. In this way, the non-objects having a diameter smaller than the opening area of the lower ends of the tapered portions 12 precipitate to the inner bottom part 2 of the container 4 without being supported by the tapered portions 12 of the through holes 8. Further, since the upper end edges of the tapered portions are pointed as shown in FIG. 3C, the selection objects 7a and the non-objects 13a are unlikely to be caught and easily introduced into the through holes 8.

On the other hand, as shown in FIG. 3D, the objects having a diameter larger than the opening area of the lower ends of the tapered portions 12 out of the collection of objects M precipitated into the through holes 8 are held in contact with the inner wall surfaces of the tapered portions 12 and the through holes 8 and supported as the selection objects 7 (selection objects 7c). FIG. 3D is a diagram of the selection objects 7 supported in contact with the tapered portions 12. Reference sign 7c denote the supported selection object. Through the above process, the collection of objects M is selected into the selection objects 7 and the non-objects 13.

The number of the through holes 8 is not particularly limited. For example, the plate 9 may include only one through hole 8 as shown in FIG. 2. In this case, FIG. 2 is also a perspective view showing a modification of the plate 9 of the first embodiment of the present invention. A plurality of through holes 8 are preferably arranged because time and labor can be drastically saved and operation efficiency can be improved as compared with the case where the selection objects 7 are individually selected.

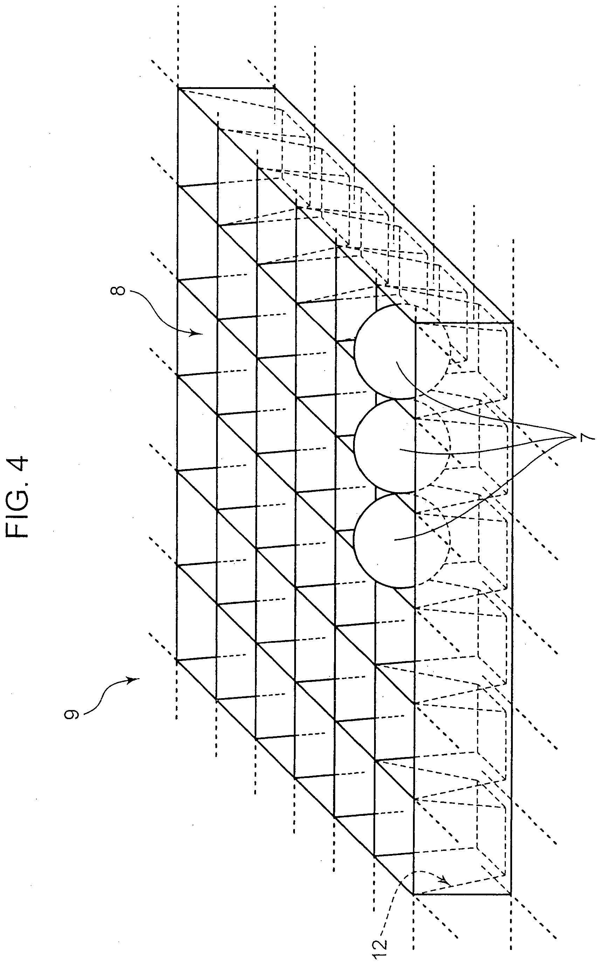

Further, as shown in FIG. 4, the plurality of through holes 8 are preferably arranged in a matrix. FIG. 4 is a perspective view showing the plate 9 in which the plurality of through holes 8 are arranged in a matrix. The number of the arranged through holes 8 is not particularly limited. In FIG. 4, the plate 9 is illustrated in which a total of thirty six through holes 8 are arranged in a 6 x 6 matrix. By forming many through holes 8 in one plate 9 in this way, the selection objects 7 having a predetermined shape can be simultaneously arranged and selected. As a result, even more selection objects 7 can be simultaneously selected as compared with the case where the selection objects 7 are individually selected and the case where the selection objects 7 are arranged in a row and selected. As a result, many selection objects can be subjected to high-throughput screening and the like and a work amount can be drastically reduced.

The shape of the through hole 8 is not particularly limited if the tapered portion 12 is formed as described above and the opening area at the upper end side of the tapered portion 12 is larger than that at the lower end side. The through hole 8 preferably has a frustum shape because the selection object 7 is easily inserted into the through hole 8 along the tapered portion 12 and because the substantially spherical selection object 7 is easily selected as compared with the case where the tapered portion 12 is provided only on a part of the inner wall surface of the through hole 8.

Types of the frustum shape may include truncated cone shapes, truncated pyramid shapes and the like. If the through hole 8 has a truncated pyramid shape, clearances are formed at the corners of the truncated pyramid in a state where the selection object 7 having a substantially spherical shape is supported by the tapered portion 12. As a result, the selection object 7 is not fitted into the through hole 8 and can be easily extracted such as by vertically inverting the plate. Above all, a truncated square pyramid shape and a truncated hexagonal pyramid shape are preferable because of easy processing and easiness to densely form many through holes 8 per unit area of the plate 9. Note that an angle of inclination of the tapered portion 12 formed in the through hole 8 needs not be equal. Further, a cross-sectional shape of the frustum shape (shape of the through hole when the plate 9 is observed from above) is not particularly limited and may have a cross-sectional shape other than a regular polygonal shape.

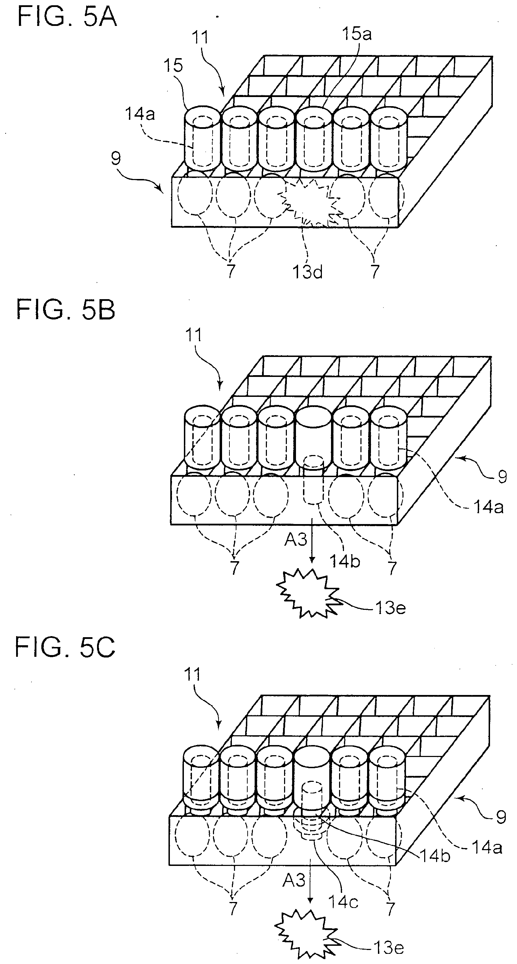

In FIG. 2, reference sign L1 denotes the length of one side of the opening at the side of the top surface 5 of the through hole 8 when the through hole 8 is formed into a truncated square pyramid shape, and reference sign L2 denotes the length of one side of the opening at the side of the bottom surface 6 of the through hole 8 when the through hole 8 is formed into a truncated square pyramid shape. A ratio (L2²/L1²) of the opening area at the side of the bottom surface 6 (L2²) to the opening area at the side of the top surface 5 (L1²) is not particularly limited, but is, for example, preferably 0.11 to 0.94, more preferably 0.17 to 0.44 and even more preferably 0.18 to 0.31. If L2²/L1² is within the above range, the plate 9, the supported selection objects 7, the non-object 13d and the push-out mechanisms 11 can be captured within a depth of field of a lens provided in the phase-contrast microscope 10 while the influence of the shadow of the plate 9 on observation is reduced when the non-object 13d is pushed out by a push-out pin 14a of the push-out mechanism 11 (see FIG. 5) to be described later while the supported selection objects 7 are observed from above or below the container 4 by the phase-contrast microscope 10. Thus, the user can easily confirm whether or not the selection objects 7 and the non-object 13d are supported by the plate 9, and the shapes of the supported selection objects 7 and non-object 13d. Further, the user can easily confirm the position of the non-object 13d and that of the push-out mechanism 11. Furthermore, even if there is a slight flow of the liquid 3 in the container 4 such as when the collection of objects M is added to the liquid 3, the selection objects 7 supported by the plate 9 are more reliably supported by the plate 9 without being flowed out of the through holes 8 by the flow.

### <Concerning Phase-Contrast Microscope 10>

The phase-contrast microscope 10 (determining means) is provided to observe the shapes of the selection objects 7 supported by the plate 9 from below the container 4. By observing objects using the phase-contrast microscope 10 in this way, the shapes can be clearly recognized and accurately observed, for example, even if the objects are bio-based cells or the like.

Note that although the phase-contrast microscope 10 is adopted as the determining means in this embodiment, the determining means is not particularly limited. Besides general optical microscopes, fluorescence microscopes, polarization microscopes, stereomicroscopes, bright-field microscopes, dark-field microscopes, differential interference microscopes, supersonic microscopes, confocal microscopes, laser scanning microscopes, electronic microscopes, scanning probe microscopes, X-ray microscopes, virtual microscopes, digital microscopes and the like can be used.

In the case of observing a selection object 7 with high transparency such as a cell, a phase-contrast microscope or a fluorescence microscope is preferably used as the determining means. In the case of using a fluorescence microscope as the determiner, an object needs to be fluorescent. Thus, in the case of measuring a non-fluorescent object by the fluorescence microscope, the object is measured after being dyed with a fluorescent dye. A method for dyeing an object is not particularly limited and a preferable dyeing method may be appropriately adopted. For example, chemical fluorescent staining, antibody fluorescent straining or a like method can be adopted. Besides, it is also possible to adopt a method for introducing a gene inducing fluorescent protein such as green fluorescent protein (GFP) into a cell through genetic recombination and observing the cell.

A monitor device (not shown) is attached to the phase-contrast microscope 10. The monitor device includes an imaging element for converting an optical image generated by the phase-contrast microscope 10 into an electrical image signal, an image processor for applying image processings such as a gamma correction and a shading correction to the image data, and a display device for displaying image data after the image processings.

Determination criteria in determining the shape of an object by the phase-contrast microscope 10 are not particularly limited. The determination criteria may be appropriately determined by the user depending on the use application of the selection object 7.

### <Concerning Push-Out Mechanism 11>

The push-out mechanisms 11 (removing means) are provided to remove the non-objects 13 determined to be defective through the image processings and analysis of the obtained image data or the like by the phase-contrast microscope 10 (determining means) by pushing out the non-objects 13 from the support positions of the plate 9.

FIGS. 5 are diagrams showing a state of operation of the push-out mechanisms 11 of this embodiment. As shown in FIG. 5A, the push-out mechanism 11 of this embodiment includes a push-out pin 14a and an actuator 15 for driving the push-out pin 14a to project downward and retract.

The push-out mechanisms 11 are arranged above the plate 9 so that the push-out pins 14a are arranged above the through holes 8 of the plate 9. FIG. 5A shows a state where the push-out mechanisms 11 each including the push-out pin 14a and the actuator 15 are arranged above six corresponding through holes 8 in the front row of the plate 9 in a state where objects are supported in the through holes 8 in the front horizontal row of the plate 9 in which a total of thirty six through holes 8 are arranged in a 6x6 matrix. Reference sign 13d denotes a supported non-object.

The actuators 15 are controlled by a drive control mechanism (not shown). The actuator 15 is provided to, if it is turned out that the non-object 13d is supported by the tapered portion 12 of the through hole 8 in an observation by the phase-contrast microscope 10, actuate the push-out pin 14a for pushing out such a non-object 13d to project downward and retract. As shown in FIG. 5A, the push-out pin 14a is housed in the actuator 15 and pushed out in response to a push-out command from the drive control mechanism.

The user causes the push-out pin 14a of the actuator 15a provided above the support position for the non-object 13d out of the actuators 15 controlled by the above drive control mechanism to project and push out the non-object 13d out of the through hole 8 when observing the objects supported at the support positions of the plate 9 using the above phase-contrast microscope 10 and finding out the object (non-object 13d) not having a predetermined shape. Specifically, out of the objects shown in FIG. 5A, the non-object 13d has a distorted shape as compared with the selection objects 7 and does not have the predetermined shape. Thus, as shown in FIG. 5B, the user causes the push-out pin 14a to project from the actuator 15 (actuator 15a) at that position to push out the non-object 13 by controlling the operation of the actuator 15 via the drive control mechanism. Reference sign 13e denotes the pushed-out non-object, reference sign 14b denotes the pushed-out push-out pin and an arrow A3 indicates a direction of projection of the push-out pin.

The push-out pin 14a is controlled by the actuator 15. The push-out pin 14a is provided to push out the non-object 13 supported in the through hole 8 through an opening at the lower end side of the tapered portion 12 (see FIG. 4) of the through hole 8.

The length of the push-out pin 14a is not particularly limited, but is preferably a length capable of sufficiently pushing out the non-object 13d supported in the through hole 8.

The shape of the push-out pin 14a is not particularly limited, but is preferably such a shape as to be vertically movable in the actuator 15 and have a large contact area with the non-object 13d because of easiness to push out the non-object 13d in pushing out the non-object 13d supported in the through hole 8. Specifically, the push-out pin 14a preferably has a cylindrical shape, a prism shape or a shape with at least a flat tip part.

As another embodiment, an appropriate jig may be mounted on the tip of the push-out pin 14a. For example, FIG. 5C is a diagram showing another example of the push-out pin 14a of this embodiment. As shown in FIG. 5C, a push-out jig 14c shaped to be suitable for pressing a non-object supported by the plate 9 is mounted on the tip of the push-out pin 14a. A projection provided on the push-out jig 14c presses and pushes out the non-object 13e in a state pushed out from the actuator 15. The shape of the push-out jig 14c is not particularly limited and may be a conical shape in conformity with the shape of the tapered portion of the through hole 8 besides the shape of the projection. The push-out jig 14c may be integrally formed to the push-out pin 14a or may be joined with the push-out pin 14a to be integral to the push-out pin 14a after being formed as a different body.

Note that six push-out mechanisms 11 arranged side by side in the front horizontal row are shown in FIGS. 5A and 5B. In the case of adopting the push-out mechanisms 11 in such an arrangement, the plate 9 or the push-out mechanisms 11 or both of them may be appropriately moved in the case of pushing out non-objects 13d supported in six through holes 8 in the second row from the front side of the plate 9.

Further, a total of thirty six actuators 15 and a total of thirty six push-out pins 14a may be arranged in a 6x6 matrix above the plate 9 in accordance with the arrangement of the through holes 8 of the plate 9. In such a case, the plate 9 or the push-out mechanisms 11 need not be moved as described above and operation efficiency can be improved.

Further, the phase-contrast microscope 10 and the push-out mechanisms 11 can be connected by an externally provided control mechanism (not shown). This enables the user to determine the objects using the phase-contrast microscope 10 and transmit position information of the non-object 13d determined to be defective to the control mechanism. The control mechanism having received the position information transmits this position information to the push-out mechanism and causes the push-out mechanism to operate at an appropriate timing.

As described above, according to the object selecting device 1 of this embodiment, only the selection objects 7 having the predetermined shape out of the collection of objects M having different shapes can be supported in the tapered portions 12 and the non-objects 13 other than the selection objects 7 can be allowed to precipitate to the inner bottom part 2 of the container 4. Further, even if the non-object 13d is supported in the tapered portion 12, the position can be confirmed through an observation by the phase-contrast microscope 10 and the non-object 13d can be pushed out and removed by the push-out mechanism 11 including the push-out pin 14a. Thus, only the selection objects 7 can be supported by the plate 9. The supported selection objects 7 need not be extracted such as by suction while causing forced deformation or the like. Thus, the selection objects 7 can be extracted by a gentle method such as collection by vertically inverting the plate 9 and suction using a suction device (not shown) with a suction tip having a suction port sufficiently larger than diameters of the selection objects 7 in such a manner as not to apply a load to the selection objects 7. Therefore, only the selection objects 7 having the predetermined shape can be selected without being deformed or destroyed.

Note that a process performed by the user in this embodiment can be automatically performed using a robot by controlling the robot by software programming the content of the process in advance.

Further, although the selection objects 7 are cell aggregates and can be gently extracted such as by vertically inverting the plate 9 after being supported by the plate 9 in this embodiment, the selection objects 7 may be kept for a predetermined time at the support positions of the plate 9 if necessary. For example, if the selection objects 7 are not sufficiently grown cell aggregates, but single cells or undergrown cell aggregates, the user can continue to cultivate these selection objects 7 kept supported at the support positions of the plate 9 and extract these selection objects 7 after the selection objects 7 have sufficiently grown. The extracted cell aggregates can be subjected to various screening.

The vertically penetrating through holes 8 are formed at the support positions of the plate 9 of this embodiment. Thus, a culture solution can be sufficiently brought into contact with undergrown cells supported at the support positions of the plate 9. As a result, the plate 9 can preliminarily select undergrown cells before being sufficiently grown and form sufficiently grown cell aggregates by cultivating the undergrown cells at the support positions.

### (Second Embodiment)

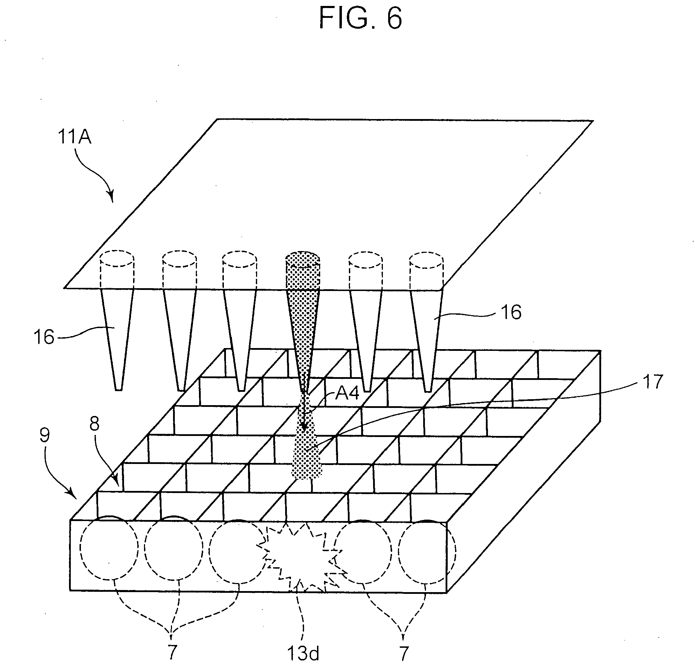

An object selecting device of a second embodiment of the present invention is described in detail below with reference to the drawing. FIG. 6 is a diagram showing a state of operation of injection nozzles 16 of the second embodiment of the present invention.

The object selecting device of the second embodiment is similar to the object selecting device 1 of the first embodiment except for including the injection nozzles 16 from which a push-out mechanism 11A (removing means, push-out means) can inject an injection liquid as shown in FIG. 6. Thus, only points of difference are described.

As shown in FIG. 6, the push-out mechanism 11A including six injection nozzles 16 arranged in a row is illustrated in this embodiment. Each injection nozzle 16 is so provided that a nozzle tip thereof is arranged above a corresponding through hole 8 in the front row of the plate 9.

The injection nozzles 16 are controlled by a drive control mechanism (not shown). If it is found out that a non-object 13d is supported in a tapered portion 12 of the through hole 8 in an observation by the phase-contrast microscope 10 described above, the injection nozzle 16 injects an injection liquid 17 for pushing out the non-object 13d.

The injection nozzles 16 receive the supply of the injection liquid 17 from an externally provided injection liquid supply source (not shown). As with the object selecting device 1 of the first embodiment, a user causes the injection nozzle 16a provided to correspond to a support position of a non-object 13d out of the injection nozzles 16 controlled by the above drive control mechanism to inject the injection liquid 17 to push out the non-object 13d from the through hole 8 when observing objects supported at support positions of the plate 9 using the phase-contrast microscope 10 and finding out the object not having a predetermined shape (non-object 13d). Specifically, as shown in FIG. 6, the user causes the injection liquid 17 to be injected from the injection nozzle (injection nozzle 16a) at that position to push out the non-object 13d by controlling the operation of the injection nozzle 16 via the drive control mechanism. An arrow A4 indicates a direction of injection of the injection liquid 17.

The shape of the nozzle tip of the injection nozzle 16 is narrowed so as to be able to inject the injection liquid 17 only to the non-object 13d and a distance between the injection nozzle 16 and the through hole 8 is so adjusted that the injection liquid is not injected to the selection objects 7 supported in the adjacent through holes 8.

Note that although the injection liquid 17 is supplied from the injection liquid supply source in this embodiment, it is preferable to provide a circulation mechanism for circulating liquid 3 stored in a container 4 to the injection nozzles 16 in the case of using the liquid 3 stored in the container 4 as the injection liquid 17. In this case, the amount of the liquid 3 stored in the container 4 is neither increased nor decreased by the injection of the injection liquid 17 by the injection nozzles 16. Further, the liquid 3 is not diluted by the injection liquid 17.

Note that the injection liquid 17 to be injected may be something other than the liquid 3 stored in the container 4 and can be appropriately selected within such a range as not to adversely affect properties of the selection objects 7. Further, gas such as air may be injected as injection gas instead of the injection liquid 17. In this case, gas which does not adversely affect the properties of the selection objects 7 can be appropriately selected as the injection gas.

As described above, according to the object selecting device of this embodiment, the non-object 13d can be pushed out by injecting the injection liquid. Thus, as compared with the case where the non-object 13d is pushed out such as by a push-out pin, there is no possibility of damaging the plate 9 since there is no contact of the push-out pin with a part of the plate 9 in removing the non-object 13d.

### (Third Embodiment)

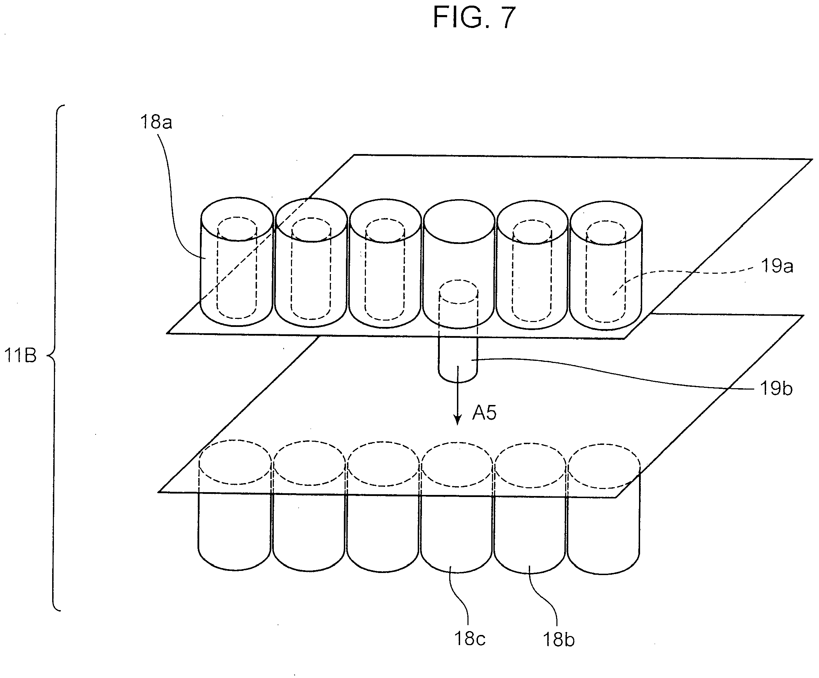

An object selecting device of a third embodiment of the present invention is described in detail below with reference to the drawing. FIG. 7 is a diagram showing a state of operation of a push-out mechanism 11B of the third embodiment of the present invention.

The object selecting device of the third embodiment is similar to the object selecting device 1 of the first embodiment except that the push-out mechanism 11B (removing means, push-out means) drives push-out pins 19a by electromagnets as shown in FIG. 7. Thus, only points of difference are described.

As shown in FIG. 7, the push-out mechanism 11B of this embodiment includes the push-out pins 19a, upper actuators 18a each with the push-out pin 19a, and lower actuators 18b each with the electromagnet for causing the push-out pin 19a to project. In the illustrated push-out mechanism 11b, six upper actuators 18a and six lower actuators 18b are arranged in a row and vertically face each other. The arrangements of the upper actuators 18a and the push-out pins 19a are similar to those of the actuators 15 and the push-out pins 14a of the first embodiment.

The lower actuators 18b are provided below a plate 9, and provided on an outer bottom part of a container 4 when the plate 9 is placed on an inner bottom part 2 of the container 4.

Note that although the plate is not shown in FIG. 7 to clearly show the operation of the push-out pins 19a in this embodiment, a positional relationship of the plate and the push-out mechanism 11B is as in the object selecting device 1 of the first embodiment.

A user causes the push-out pin 19a included in the upper actuator 18a provided to correspond to a position where a non-object is supported out of the upper actuators 18a to project and push out the non-object 13 from a through hole by energizing the electromagnet controlled by a drive control mechanism when observing objects supported at support positions of the plate 9 using the phase-contrast microscope 10 described above and finding out the object not having a predetermined shape (non-object). Specifically, as shown in FIG. 7, the push-out pins 19a are housed in the upper actuators 18a. In a state where the electromagnets of the lower actuators are not energized (lower actuators 18b), the push-out pins 19a remain housed in the upper actuators 18a. On the other hand, in the case of energizing the electromagnet of the lower actuator (lower actuator 18c), the push-out pin 19a (push-out pin 19b) projects from the upper actuator 18a, thereby allowing the non-object to precipitate to the inner bottom part of the container through an opening at the lower end of a tapered portion 12. An arrow A5 indicates a direction of projection of the push-out pin 19a.

Note that although the lower actuators 18b each with the electromagnet are provided below the plate 9 in FIG. 7, the positions of actuators each with an electromagnet are not particularly limited. For example, if actuators each with an electromagnet are provided further above the upper actuators 18a and energized in a direction opposite to a direction of energization to the electromagnets of the lower actuators 18b, the push-out pins 19a can be pushed out from the upper actuators 18a. By providing the actuators each with the electromagnet further above the upper actuators 18a in this way, the user can more easily observe the states of the object from below the container using the phase-contrast microscope 10, wherefore convenience such as operability is improved.

Further, an elastic member such as a coiled compression spring may be provided in each upper actuator 18a and the push-out pin 19a may be controlled to project and retract. Specifically, the user can control the push-out pin 19a in such a manner that the push-out pin 19a is held in the upper actuator 18a while compressing the compression spring, for example, by energizing the electromagnet. In this case, the user can cause the push-out pin 19a to be pushed out from the upper actuator 18a utilizing an elastic force of the compression spring by stopping energization to the electromagnet only in pushing out the push-out pin 19a from the upper actuator 18a.

### (Fourth Embodiment)

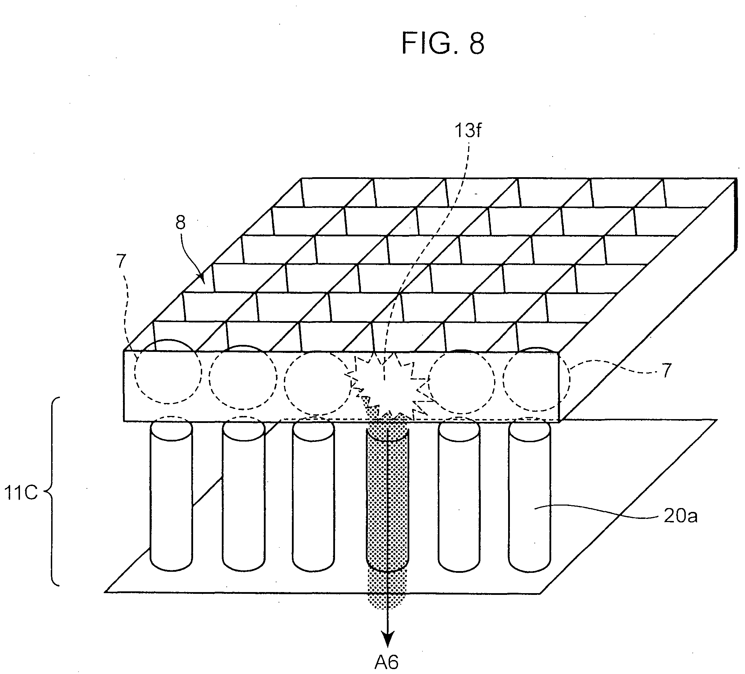

An object selecting device of a fourth embodiment of the present invention is described in detail below with reference to the drawing. FIG. 8 is a diagram showing a state of operation of a pull-out mechanism 11 C (removing means, pull-out means) of the fourth embodiment of the present invention.

The object selecting device of the fourth embodiment is as in the first embodiment except that the pull-out mechanism 11C including suction nozzles 20a is provided as a removing means on the side of a bottom surface 6 of a plate 9 as shown in FIG. 8. Thus, only points of difference are described.

As shown in FIG. 8, the suction nozzle 20a is provided near an opening at a lower end side of a tapered portion of each through hole 8. The suction nozzles 20a are arranged in a row below the plate 9 and correspond to the respective through holes 8. The suction nozzles 20a are nozzles for sucking objects supported in the tapered portions. In FIG. 8, reference sign 20a denotes the suction nozzle in a non-suction state, reference sign 20b denotes the suction nozzle in a suction state, and reference sign 13f denotes a sucked non-object 13.

The suction nozzle 20a includes a valve mechanism (not shown) controlled by a drive control mechanism (not shown), and is connected to an externally provided suction device (not shown) via the valve mechanism. If it is found out that the non-object 13f is supported in the tapered portion of the through hole 8 in an observation by the phase-contrast microscope 10 described above, the user drives the external suction device and the drive control mechanism to open only the valve mechanism of the corresponding suction nozzle 20a, thereby sucking the non-object 13f to pull out and remove the non-object 13f from the through hole 8. An arrow A6 indicates a direction of pulling out the non-object 13f by the suction nozzle 20b.

The tip of the suction nozzle 20a is provided at a position sufficiently close to the corresponding through hole 8 so as not to erroneously pull out selection objects 7 supported in the tapered portions of the adjacent through holes 8.

As described above, according to the object selecting device of this embodiment, since the non-object 13f can be pulled out and removed, there is no contact with a part of the plate in removing the non-object 13f as compared with the case where the non-object 13f is pushed out such as by a push-out pin. Thus, there is no possibility of damaging the plate. Further, if a position where the plate is to be placed is determined in the container, the pull-out mechanism 11C including the suction nozzles 20a has only to be provided at a position corresponding to the plate to be placed, wherefore operation efficiency can be drastically improved.

Note that although the suction nozzles 20a are arranged in a row below the plate 9 in this embodiment, the number of the arranged suction nozzles 20a is not particularly limited. One suction nozzle 20a may be arranged or a plurality of suction nozzles 20a may be arranged in a row. Suction nozzles 20a may be, for example, arranged in a 6x6 matrix in accordance with the positions of the through holes 8.

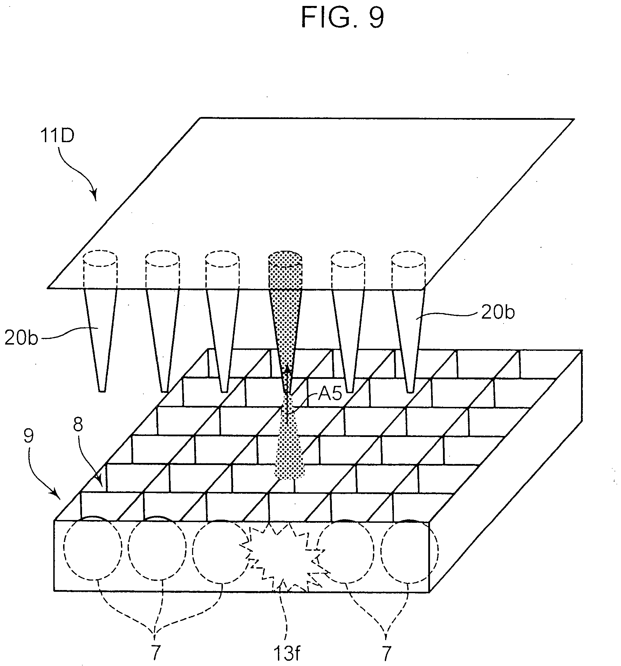

Further, the arranged positions of the suction nozzles 20a are not particularly limited. The suction nozzles 20 may be arranged above the plate 9 as shown in FIG. 9. FIG. 9 is a diagram showing a state of operation of a modification of the pull-out mechanism 11C (pull-out mechanism 11D) of this embodiment. As shown in FIG. 9, suction nozzles 20b arranged above are connected to an externally provided suction device (not shown) and suck the non-object 13f by operating the suction device and generating a suction force at a suction port of the suction nozzle 20b. An arrow A5 indicates a direction of sucking the non-object 13f by the suction nozzle 20b. The pulled-out non-object 13f is sucked by the suction nozzle 20b and discarded to a disposal space connected to an inner passage in the suction nozzle 20b through the inner passage.

### [Object Selection Method]

Next, the object selecting method of this embodiment is described. The object selecting method of this embodiment is an object selecting method for selecting selection objects from a collection of objects including the selection objects, and includes an immersion step, a precipitation step, an arrangement step, a determination step and a removal step. Each step is described below.

### <Concerning Immersion Step>

The immersion step is a step of immersing a plate having a top surface and a bottom surface and configured to support selection objects in a container including an inner bottom part and storing liquid. The container and the plate are not described since being the same as those described in detail in the description of the object selecting device 1 of the first embodiment.

As shown in FIG. 3A, the plate 9 is immersed in the container 4 storing the liquid 3. FIG. 3A is a diagram of the plate 9 to be immersed into the container 4 storing the liquid 3. In this way, the plate 9 is immersed into the liquid 3 in advance in a state where nothing is supported at the support positions. This enables the user to select the selection objects 7 in the liquid 3 and prevent the selection objects 7 included in the collection of objects M (see FIG. 3B) such as a cell culture solution from being exposed to outside air and being dried.

### <Concerning Precipitation Step and Arrangement Step>

The precipitation step is a step of adding the collection of objects including the selection objects to the liquid from a side of the top surface of the plate and causing the collection of objects to precipitate along the direction of gravity into through holes formed in the plate, arranged at support positions where the selection objects are supported and including tapered portions on the inner wall surfaces thereof, the opening area at the upper ends of the tapered portions being larger than that at the lower ends of the tapered portions.

Further, the arrangement step is a step of bringing the selection objects out of the collection of objects precipitating in the through holes into contact with the tapered portions and supporting them at the support positions.

The collection of objects and the through holes are not described since being the same as those described above. A procedure of selecting the precipitating selection objects and arranging them at the support positions of the plate is described below. FIGS. 3B to 3D are diagrams showing a procedure of selecting the selection objects 7 in the object selecting device 1.

As shown in FIG. 3B, the collection of objects M is added to the liquid 3 from the side of the top surface of the plate 9 immersed in the liquid 3. FIG. 3B is a diagram showing a state where the collection of objects M including the selection objects 7 is added to the liquid 3 from the side of the top surface of the plate 9.

A method for adding the collection of objects M is not particularly limited, but the collection of objects M is preferably gently added from a position close to the liquid level or directly added to the liquid 3 using a pipette or the like when being added to the liquid 3 in order to eliminate the drying of the selection objects 7 and a physical impact. The collection of objects M added to the liquid 3 gently precipitates with gravity while being dispersed in the liquid 3. Thus, a physical impact on the selection objects 7 is reduced.

Subsequently, as shown in FIG. 3C, the collection of objects M precipitates in the liquid 3 with gravity and reaches the top surface of the plate 9. FIG. 3C is a diagram showing a state where the collection of objects M added to the liquid 3 precipitates in a plurality of arranged through holes 8 of the plate 9 along the direction of gravity. As described above, out of the collection of objects M having reached the top surface of the plate 9 with gravity, the selection objects 7 and the non-objects 13 in contact with the tapered portions 12 are introduced into the through holes 8 while descending along the tapered portions 12.

Since the diameters of the non-objects 13 are smaller than the opening area at the lower ends of the tapered portions 12, the non-objects 13 pass through the through holes 8. The non-objects 13 having passed through the through holes (non-objects 13b) precipitate to the inner bottom part 2 of the container.

On the other hand, as shown in FIG. 3D, the objects (selection objects 7c) having larger diameters than the opening area at the lower ends of the tapered portions 12 out of the collection of objects M precipitating into the through holes 8 are supported by the tapered portions 12 in contact with the inner wall surfaces of the tapered portions 12 and the through holes 8 and arranged at the support positions of the plate 9.

### <Concerning Determination Step>

The determination step is a step of determining whether or not the objects supported at the support positions of the plate through the precipitation step and the arrangement step have a predetermined shape.

A determination method is not particularly limited, and a method for observation using the phase-contrast microscope (determining means) described above or the like can be adopted. The objects determined to be defective (non-objects) in this determination step are removed by a removing means such as the push-out mechanism in the removal step to be described next.

### <Concerning Removal Step>

The removal step is a step of removing the objects determined to be defective in the determination step by the push-out mechanism (removing means).

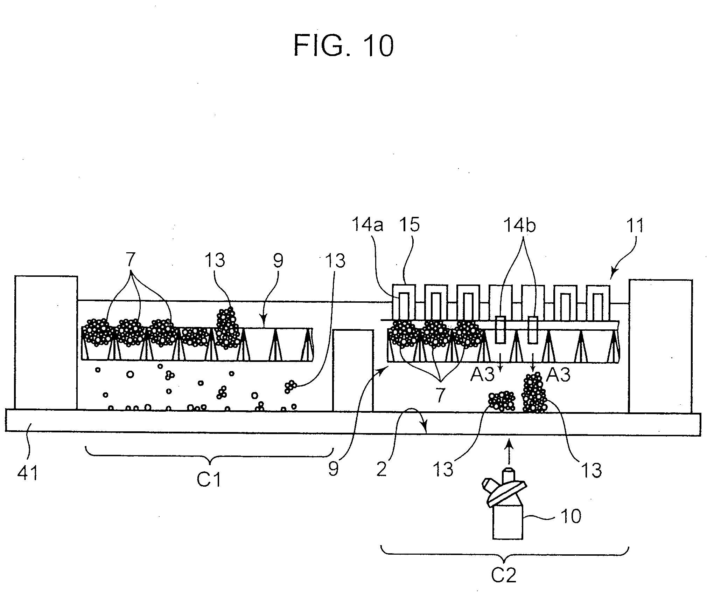

A container provided with the push-out mechanism is described in more detail with reference to FIG. 10. FIG. 10 is a diagram showing each step of the object selecting method of this embodiment. In FIG. 10, a container 41 divided into two chambers is used.

As shown in FIG. 10, in a cell capture chamber C1, the selection objects 7 described above are selected and the objects having different diameters are selected and removed. A state of selection is obtained mainly by observing the presence or absence of the objects supported by the plate 9 by the phase-contrast microscope 10 (determining means) provided above or below the container 41. At this point of time, the non-objects 13 having diameters smaller than the opening area at the lower ends of the tapered portions are removed and the selection objects 7 are selected in the tapered portions. However, at this point of time, there is a possibility that objects having larger diameters or having distorted shapes are supported by the plate 9 besides the objects having a desired shape. Thus, the plate 9 is moved to an adjacent cell selection chamber C2 and further selection proceeds based on the shape.

In the cell selection chamber C2, the shapes of the objects supported by the plate 9 are observed by the phase-contrast microscope 10 provided above or below the container. Besides the phase-contrast microscope 10, the fluorescence microscope described above or the like can be adopted as the determining means. The phase-contrast microscope 10 is used by being appropriately moved from a position of observation in the cell capture chamber C1, but the phase-contrast microscopes 10 corresponding to the respective chambers may be prepared. Further, the position of the phase-contrast microscope 10 may be fixed and the container 41 may be moved.

By adopting the phase-contrast microscope 10 as the determining means, objects which are substantially transparent can be clearly observed. Further, if the selection objects 7 are dyed with a fluorescent dye such as trypan blue, they can be observed by the fluorescence microscope.

If the selection objects 7 are, for example, cell aggregates, the user observes the objects supported in the tapered portions of the plate using the phase-contrast microscope 10. As a result, if the object has a diameter larger than a predetermined diameter or a distorted shape other than the predetermined shape or is a defective cell aggregate including dead cells, the user can newly determine this object as the defective non-object 13. The non-object 13 determined to be defective is removed by the push-out mechanism 11 (removing means, push-out means) described above.

Thereafter, the selection objects 7 are collected by a gentle method such as by vertically inverting the plate 9 or by sucking the selection objects 7 in such a manner as not to apply a load to the selection objects 7 using a suction device (not shown) with a suction tip including a suction port sufficiently larger than the diameters of the selection objects 7.

Note that although the push-out mechanisms 11 described in detail in the first embodiment are adopted for a removing method in the object selecting method described above, the removing method is not particularly limited. Removing methods using various removal mechanisms (removers) can be adopted as well as the push-out mechanisms and the pull-out mechanisms shown in the second to fourth embodiments. Further, the user can also adopt a method for removing the non-object 13 by irradiating laser light. For example, the user can remove the non-object 13 by confirming the position of the non-object 13 supported by the plate 9 using the phase-contrast microscope 10 or the like and fracturing the non-object 13 by irradiating laser light to the non-object 13 or causing apoptosis or necrosis. An ultraviolet laser having a wavelength of 350 nm or a green semiconductor laser having a wavelength of 532 nm can be, for example, used as the laser light to be irradiated.

As described above, according to the object selecting method of this embodiment, it is possible to support only the selection objects having the predetermined shape out of the collection of objects having different shapes by the plate and allow the non-objects having smaller particle diameters than the predetermined shape to precipitate to the inner bottom part of the container. Further, whether or not the non-object having a distorted shape is included in the objects supported by the plate can be determined by the determining means and, if such a non-object is present, it can be removed by the removing means. As a result, only the selection objects can be supported by the plate. Since the supported selection objects need not be extracted such as by suction while causing forced deformation or the like and applying a load to the objects, they can be gently extracted such as by a method of vertically inverting the plate. Thus, only the selection objects having the predetermined shape can be selected without being deformed or destroyed.

Note that the aforementioned specific embodiments mainly include inventions having the following configurations.

An object selecting device according to one aspect of the present invention is an object selecting device as described above.

In the present invention, by adopting such a configuration, only the selection object having a predetermined shape out of the collection of objects having different shapes is supported by the plate and non-objects having smaller particle diameters than the predetermined shape are allowed to precipitate to the inner bottom part of the container. Further, whether or not any non-object having a distorted shape is included in the objects supported by the plate is determined by the determining means. If such a non-object is present, it can be removed by the removing means. As a result, only the selection object can be supported by the plate. Since the supported selection object needs not be extracted such as by suction while causing forced deformation or the like, it can be gently extracted by a method such as collection by vertically inverting the plate or suction in such a manner as not to apply a load to the selection object using a suction device with a suction tip including a suction port sufficiently larger than a diameter of the selection object. Thus, only the selection object having the predetermined shape can be selected without being deformed or destroyed.

According to the present invention, the through hole includes a tapered portion which allows the selection object to precipitate along a direction of gravity and supports the selection object by bringing the selection object into contact with the inner wall surface of the through hole in a state where the plate is immersed in the liquid in the container, and an opening area at the upper end of the tapered portion is larger than an opening area at the lower end of the tapered portion.

In the present invention, by adopting such a configuration, the collection of objects added from above the plate is easily introduced into the through hole via the tapered portion when reaching the top surface of the plate.

The through hole preferably has a frustum shape.

In the present invention, by adopting such a configuration, the selection object is easily introduced into the through hole since the inner side surface of the through hole serves as the inclined tapered portion. Further, the selection object having a substantially spherical shape is easily selected, for example, as compared with the case where the tapered portion is provided on a part of the inner wall surface of the through hole.

According to the present invention, the plate preferably includes a plurality of the arranged through holes.

In the present invention, by adopting such a configuration, selection objects having a plurality of predetermined shapes can be simultaneously selected and labor and time can be drastically saved and operation efficiency can be improved as compared with the case of individually selecting the selection objects. If a plurality of nozzles are prepared in correspondence with the arrangement of the through holes, for example, in the case of extracting the selection objects by nozzles or the like, it can contribute to the automation of the device. Note that the plurality of nozzles can be moved in accordance with the arrangement of the through holes.

The plate preferably includes a plurality of the through holes arranged in a matrix.

In the present invention, by adopting such a configuration, more selection objects can be simultaneously selected. As a result, selection objects can be subjected to high-throughput screening and the like and a work amount can be drastically reduced.

The container and the plate are preferably made of a translucent material.

In the present invention, by adopting such a configuration, the selection objects can be continuously confirmed such as by a microscope from above or below the container and operation efficiency can be improved.

The determining means is preferably a phase-contrast microscope.

In the present invention, by adopting such a configuration, even if a bio-based cell or the like is the selection object, the shape can be clearly recognized and accurate selection can be made.

Preferably, the collection of objects is dyed with a fluorescent dye and the determining means is a fluorescence microscope.

In the present invention, by adopting such a configuration, even if the selection object whose shape is difficult to confirm with the naked eye or another observation method, the shape can be clearly recognized and accurate selection can be made.

The removing means is preferably a push-out means for removing the object determined to be defective by the determining means by pushing out the object from the through hole.

In the present invention, by adopting such a configuration, the non-object supported by the plate can be reliably caused to drop to the inner bottom part of the container. Further, since the non-object is pushed out from the upper end side to the lower end side of the tapered portion, it can be pushed out while being observed by the determining means, wherefore operation efficiency is improved.

The removing means is preferably a pull-out means for removing the object determined to be defective by the determining means by pulling out the object from the through hole.

In the present invention, by adopting such a configuration, the object can be removed by being sucked together with the surrounding liquid. Thus, a possibility that a part of the non-object remains without being removed is reduced and removal efficiency can be improved.

The selection object is preferably a bio-based cell.

By adopting such a configuration, the present invention can be applied to the bio-based cell, which is an object with a large shape deviation, and can provide a device capable of contributing to an improvement in operation efficiency in the fields of bio-related technology and medicine.

The selection object is preferably a bio-related cell aggregate.

The bio-based cell aggregate can provide a result considering functions of individual cells as compared with a test result obtained using one cell since a biosimilar environment considering interactions among cells is reconfigured in the cell aggregate, and experiment conditions can be made uniform in accordance with an environment in a biological body. Thus, by adopting such a configuration, the present invention can provide a device capable of obtaining a highly reliable result in the fields of bio-related technology and medicine.

An object selecting method according to another aspect of the present invention is an object selecting method as described above.

In the present invention, by adopting such a configuration, only the selection object having a predetermined shape out of the collection of objects having different shapes is supported by the plate and non-objects having smaller particle diameters than the predetermined shape are allowed to precipitate to the inner bottom part of the container. Further, whether or not any non-object having a distorted shape is included in the objects supported by the plate is determined by the determiner. If such a non-object is present, it can be removed by the remover. As a result, only the selection object can be supported by the plate. Since the supported selection object needs not be extracted such as by suction while causing forced deformation or the like, it can be gently extracted by a method such as collection by vertically inverting the plate. Thus, only the selection object having the predetermined shape can be selected without being deformed or destroyed.

## Claims

1. An object selecting device (1) for selecting a selection object (7) from a collection of objects including the selection object (7), comprising:
a container (4) including an inner bottom part (2) and configured to store liquid (3);
a plate (9) having a top surface (5) and a bottom surface (6), including a plurality of through holes (8) at a support position for the selection object (7), each of the through holes (8) penetrating from the top surface (5) to the bottom surface (6), and to be immersed in the liquid (3) stored in the container (4);
a determining means (10) arranged below the container (4) and including a microscope for observing from below the container (4) whether or not the object supported at the support position is desired; and
a removing means (11) arranged above the container (4) and configured to remove the object determined to be defective by the determining means (10) and leaving only the selection object (7) in a state supported at the support position, **characterized in that**;
the through hole (8) includes tapered walls that form pointed portions between tapered portions (12) of the through hole (8) such that when an object immersed in a liquid precipitates under gravity, it is brought into contact with the inner wall surface of the through hole (8), and an opening area at the upper end of the tapered portion is larger than an opening area at the lower end of the tapered portion (12),
the determining means (10) observes the objects supported at the support position, the supported objects having a size allowing to be introduced into the through hole (8) from the upper ends of the tapered portions (12) while not allowing to pass through the lower ends of the tapered portions (12), for determining whether or not the objects are desired, and
the removing means (11) removes the objects determined to be defective by the determining means (10) out of the objects supported by the inner wall surface (12).

2. An object selecting device (1) according to claim 1, wherein the container (4) and the plate (9) are made of a translucent material.

3. An object selecting device (1) according to claim 1 or 2, wherein the microscope is a phase-contrast microscope.

4. An object selecting device (1) according to any one of claims 1 to 3, wherein the collection of objects is dyed with a fluorescent dye, and the microscope is a fluorescence microscope.

5. An object selecting device (1) according to any one of claims 1 to 4, wherein the selection object (7) is a bio-based cell.

6. An object selecting device (1) according to claim 5, wherein the selection object (7) is a bio-related cell aggregate.

7. An object selecting method for selecting a selection object (7) from a collection of objects including the selection object (7), by using the object selecting device (1) according to any one of claims 1 to 6, comprising:
an immersion step of immersing the plate (9) in the container (4) storing liquid (3);
a precipitation step of adding a collection of objects including the selection object (7) to the liquid (3) from a side of the top surface (5) of the plate (9) and causing the collection of objects to precipitate along a direction of gravity into the through hole (8);
an arrangement step of supporting objects having a size allowing to be introduced into the through hole (8) from the upper ends of the tapered portions (12) while not allowing to pass through the lower ends of the tapered portions (12) out of the collection of objects precipitating in the through hole (8) at the support position;
a determination step of determining from below the container (4) by a microscope included in a determining means (10) whether or not the object supported at the support position is desired; and
a removal step of removing the object detected to be defective by the determining means (10) by a removing means (11) arranged above the container (4) and leaving only the selection object (7) in a state supported at the inner wall surface out of the objects supported by the support position.

## Patentansprüche

1. Objektauswahlvorrichtung (1) zum Auswählen eines Auswahlobjektes (7) aus einer Sammlung von Objekten, das Auswahlobjekt (7) einschließend, umfassend:
einen Behälter (4), einschließend ein inneres Unterteil (2) und gestaltet, um Flüssigkeit (3) aufzubewahren;
eine Platte (9) mit einer oberen Oberfläche (5) und einer unteren Oberfläche (6), einschließend eine Mehrzahl von Durchgangslöchern (8) an einer Trageposition für das Auswahlobjekt (7), wobei jedes der Durchgangslöcher (8) von der oberen Oberfläche (5) zu der unteren Oberfläche (6) durchgeht, und um in die Flüssigkeit (3), aufbewahrt in dem Behälter (4), eingetaucht zu werden;
ein Bestimmungsmittel (10), angeordnet unter dem Behälter (4) und einschließend ein Mikroskop zur Beobachtung von unterhalb des Behälters (4), ob das Objekt, getragen an der Trageposition, gewünscht ist oder nicht; und
ein Entfernungsmittel (11), angeordnet über dem Behälter (4) und gestaltet, um das Objekt, das von dem Bestimmungsmittel (10) als schadhaft bestimmt ist, zu entfernen und nur das Auswahlobjekt (7) in einem Zustand, getragen an der Trageposition, zurückzulassen, **dadurch gekennzeichnet, dass**;
das Durchgangsloch (8) konische Wände, die spitze Bereiche zwischen konischen Bereichen (12) des Durchgangslochs (8) bilden, einschließt, so dass, wenn ein Objekt, eingetaucht in eine Flüssigkeit, sich durch Schwerkraft absetzt, es mit der inneren Wandoberfläche des Durchgangslochs (8) in Kontakt gebracht wird, und ein Öffnungsbereich an dem oberen Ende des konischen Bereichs größer ist als ein Öffnungsbereich am unteren Ende des konischen Bereichs (12),
das Bestimmungsmittel (10) die Objekte, getragen an der Trageposition, beobachtet, wobei die getragenen Objekte eine Größe aufweisen, die es erlaubt, in das Durchgangsloch (8) von den oberen Enden der konischen Bereiche (12) eingebracht zu werden, während sie es nicht erlaubt, durch die unteren Enden der konischen Bereiche (12) zu gelangen, um zu bestimmen, ob die Objekte gewünscht sind oder nicht, und
das Entfernungsmittel (11) die Objekte, die von dem Bestimmungsmittel (10) als schadhaft bestimmt wurden, aus den Objekten, getragen von der inneren Wandfläche (12), entfernt.

2. Objektauswahlvorrichtung (1) nach Anspruch 1, wobei der Behälter (4) und die Platte (9) aus einem lichtdurchlässigen Material hergestellt sind.

3. Objektauswahlvorrichtung (1) nach Anspruch 1 oder 2, wobei das Mikroskop ein Phasenkontrastmikroskop ist.

4. Objektauswahlvorrichtung (1) nach einem der Ansprüche 1 bis 3, wobei die Sammlung von Objekten mit einem Fluoreszenzfarbstoff gefärbt ist und das Mikroskop ein Fluoreszenzmikroskop ist.

5. Objektauswahlvorrichtung (1) nach einem der Ansprüche 1 bis 4, wobei das Auswahlobjekt (7) eine bio-basierte Zelle ist.

6. Objektauswahlvorrichtung (1) nach Anspruch 5, wobei das Auswahlobjekt (7) ein bio-bezogenes Zellaggregat ist.

7. Objektauswahlverfahren zur Auswahl eines Auswahlobjektes (7) von einer Sammlung von Objekten, einschließend das Auswahlobjekt (7), durch Verwenden der Objektauswahlvorrichtung (1) nach einem der Ansprüche 1 bis 6, umfassend:
einen Eintauchschritt des Eintauchens der Platte (9) in den Flüssigkeit (3) aufbewahrenden Behälter (4);
einen Absetzschritt des Zugebens einer Sammlung von Objekten, einschließend das Auswahlobjekt (7), zu der Flüssigkeit (3) von einer Seite der oberen Oberfläche (5) der Platte (9) und des Veranlassens des Absetzens der Sammlung von Objekten entlang einer Schwerkrafts-Richtung in das Durchgangsloch (8);
einen Anordnungsschritt des Tragens von Objekten mit einer Größe, die es erlaubt, in das Durchgangsloch (8) von den oberen Enden der konischen Bereiche (12) eingebracht zu werden, während sie es nicht erlaubt, durch die unteren Enden der konischen Bereiche (12) aus der Sammlung von Objekten, abgesetzt in dem Durchgangsloch (8) an der Trageposition, zu gelangen;
einen Bestimmungsschritt des Bestimmens von unterhalb des Behälters (4) durch ein Mikroskop, eingeschlossen in einem Bestimmungsmittel (10), ob das Objekt, getragen an der Trageposition gewünscht ist oder nicht; und
einen Entfernungsschritt des Entfernens des durch das Bestimmungsmittel (10) als schadhaft erkannten Objekts durch ein Entfernungsmittel (11), angeordnet über dem Behälter (4), und des ausschließlichen Zurücklassens des Auswahlobjekts (7) in einem Zustand, getragen an der inneren Wandoberfläche, von den Objekten, getragen an der Trageposition.

## Revendications

1. Dispositif de sélection d'objet (1) destiné à sélectionner un objet à sélectionner (7) à partir d'une collection d'objets comportant l'objet à sélectionner (7), comprenant :
un conteneur (4) comportant une partie inférieure interne (2) et configuré de manière à stocker du liquide (3) ;
une plaque (9) présentant une surface supérieure (5) et une surface inférieure (6) comportant une pluralité d'orifices traversants (8) à une position de support de l'objet à sélectionner (7), chacun des orifices traversants (8) pénétrant de la surface supérieure (5) à la surface inférieure (6), et devant être immergée dans le liquide (3) stocké dans le conteneur (4) ;
un moyen de détermination (10), agencé au-dessous du conteneur (4) et comportant un microscope destiné à observer depuis le dessous du conteneur (4) si l'objet supporté à la position de support est ou non souhaité ; et
un moyen d'enlèvement (11) agencé au-dessus du conteneur (4) et configuré de manière à enlever l'objet déterminé comme étant défectueux par le moyen de détermination (10) et à laisser uniquement l'objet à sélectionner (7) dans un état supporté à la position de support, **caractérisé en ce que** :
l'orifice traversant (8) comporte des parois coniques qui forment des parties effilées entre les parties coniques (12) de l'orifice traversant (8) de telle sorte que, lorsqu'un objet immergé dans un liquide précipite sous l'action de la gravité, il est amené en contact avec la surface de paroi interne de l'orifice traversant (8), et une section débouchant à l'extrémité supérieure de la partie conique est plus grande qu'une section débouchant à l'extrémité inférieure de la partie conique (12),
le moyen de détermination (10) observe les objets supportés à la position de support, les objets supportés présentant une taille leur permettant d'être introduits dans l'orifice traversant (8) à partir des extrémités supérieures des parties coniques (12) tout en n'autorisant pas le passage à travers les extrémités inférieures des parties coniques (12), afin de déterminer si les objets sont ou non souhaités, et
le moyen d'enlèvement (11) enlève les objets déterminés comme étant défectueux par le moyen de détermination (10), excepté les objets supportés par la surface de paroi interne (12).

2. Dispositif de sélection d'objet (1) selon la revendication 1, dans lequel le conteneur (4) et la plaque (9) sont réalisés en un matériau translucide.

3. Dispositif de sélection d'objet (1) selon la revendication 1 ou 2, dans lequel le microscope est un microscope à contraste de phase.

4. Dispositif de sélection d'objet (1) selon l'une quelconque des revendications 1 à 3, dans lequel la collection d'objets est colorée avec un colorant fluorescent et le microscope est un microscope à fluorescence.

5. Dispositif de sélection d'objet (1) selon l'une quelconque des revendications 1 à 4, dans lequel l'objet à sélectionner (7) est une cellule à base biologique.

6. Dispositif de sélection d'objet (1) selon la revendication 5, dans lequel l'objet à sélectionner (7) est un agrégat de cellules associées biologiquement.

7. Procédé de sélection d'objet destiné à sélectionner un objet à sélectionner (7) à partir d'une collection d'objets comportant l'objet à sélectionner (7), en utilisant le dispositif de sélection d'objet (1) selon l'une quelconque des revendications 1 à 6, comprenant :
une étape d'immersion consistant à immerger la plaque (9) dans le conteneur (4) stockant un liquide (3) ;
une étape de précipitation consistant à ajouter au liquide (3) une collection d'objets comportant l'objet à sélectionner (7) à partir d'un côté de la surface supérieure (5) de la plaque (9) et à amener la collection d'objets à se précipiter suivant le sens de la gravité dans l'orifice traversant (8) ;
une étape d'agencement consistant à supporter des objets présentant une taille leur permettant d'être introduits dans l'orifice traversant (8) à partir des extrémités supérieures des parties coniques (12) tout n'en autorisant pas leur passage à travers les extrémités inférieures des parties coniques (12) excepté la collection d'objets précipitant dans l'orifice traversant (8) à la position de support ;
une étape de détermination consistant à déterminer, à partir du dessous du conteneur (4), par un microscope contenu dans un moyen de détermination (10), le fait que l'objet supporté à la position de support est ou non souhaité ; et
une étape d'enlèvement consistant à enlever l'objet détecté comme étant défectueux par le moyen de détermination (10) par un moyen d'enlèvement (11) agencé au-dessus du conteneur (4) et à laisser uniquement l'objet à sélectionner (7) dans un état supporté au niveau de la surface de paroi interne, hors des objets supportés par la position de support.
